# EUROPEAN PATENT SPECIFICATION

(11) **EP 1 669 102 B1**
(45) Date of publication and mention of the grant of the patent: **07.05.2008**
(21) Application number: 05110980.9
(22) Date of filing: 18.11.2005
(51) Int. Cl.: A61N 5/067, A61N 5/06

(54) **Equipment for laser treatment**
Lasertherapieeinrichtung
Equipement de traitement laser

(30) Priority: 07.12.2004 IT GE20040110
(43) Date of publication of application: 14.06.2006
(73) Proprietor: R.G.M. S.p.A., 16153 Genova (IT)
(72) Inventor: BARATTO, Luigi, 16167, GENOVA (IT); CAPRA, Roberto, 17100, SAVONA (IT); GALLAMINI, Michele, 16167, GENOVA (IT); CARTABIANCA, Luca, 16152, GENOVA (IT)
(74) Representative: Porsia, Attilio

(56) References cited:
- EP-A- 1 106 209
- DE-A1- 3 226 507
- DE-U1- 29 613 714
- US-A- 4 232 678
- US-A- 4 572 189
- US-A- 5 409 482

## Description

This invention relates to equipment for laser treatment.

Equipments of this kind comprise devices capable of emitting coherent light radiation in the range between the red and infrared (600 - 1500 nm); its use in the treatment of both acute and chronic diseases is well known. In particular, laser treatment is used when it is necessary to increase speed of recovery in the repair of damaged tissues, provide relief in the case of painful syndromes, or reduce inflammation.

Laser treatment, and more generally the means of treatment of so-called "physical medicine" have been the subject of intense debate, mainly regarding the complete lack of any comparison for the therapeutic capabilities of these means, for a long time. All in all, on the basis of information in the scientific literature it is not possible to either confirm or deny the validity of physical treatments. It is in fact evident that, despite being discontinuous and not very repeatable, physical treatments can provide benefit to the patient, but it is also evident that these do not provide the constancy of results which would render their validity incontrovertible.

From an examination of the investigations carried out into this subject so far two fundamental considerations arise:
(1) the efficacy of the physical means does not lie so much in the energy used but in its capacity to stimulate physiological responses through signals carried to the patient's body at different energies.
(2) dysfunctions treated by physical treatments have common features regardless of the disease from which they derive.

Reflex therapy and acupuncture techniques merit particular mention. These two are practices which use stimulation with an undoubted beneficial result. The nature product of an empirical approach which has been developed over the course of more than 5,000 years of observation, traditional Chinese medicine uses peripheral stimulation to activate biological response mechanisms which in propagating along particular channels known as meridians promote the therapeutic effect. Mechanical stimulation (needles) or thermal stimulation (moxibustion) act on particular points known as acupuncture points, which have special morphological and histological characteristics, as well as special biophysical anomalies, such as for example electrical conductivity and optical transparency. Notwithstanding the scepticism of the western medical profession, the World Health Organization has considered acupuncture to be valid in the treatment of many diseases.

The mechanism of transducing the peripheral stimulus is not yet wholly clear, neither is the mechanism of communication of the signal along the Meridians clear, nor finally is the mechanism of the body's response to stimulation which is capable of inducing beneficial effects clear. Among the effects of stimulation changes in the state of the extracellular matrix of soft connective tissue and some curious paraesthetic effects which are difficult to explain purely through segmental metamerization mechanisms have however been observed. Neurochemical changes have also been found in the medulla and ganglia, and through the use of modern techniques (functional PET, NMR) cortical reflexes to the stimulation of acupuncture points have been verified. Poor or insufficient understanding of the biological mechanisms activated, despite the evidence confirming the efficacy of acupuncture, is perhaps the main cause of the wholly marginal role which it has been recognized in Western clinical practice.

Efforts at cultural mediation and modelling acupuncture on the basis of the criteria of western medicine have always had an important and obvious role in the promotion of reflexotherapy methods, which, although having an almost constant common matrix of oriental origin (Shiatzu, Yoga, Ayurveda, plantar reflexology, etc.), through Western rationalization are spreading with various degrees of success into American and European medical practice. Again in this case the benefits are incontrovertible, but the explanatory models are not at all convincing.

All in all, it should be borne in mind that:
a) there are projection mechanisms, particularly for nociceptive stimuli, which although extensively observed by conventional medical semiotics have not yet found unanimously accepted explanatory models,
b) there are reasons for assuming that there is an interaction, although one which is not yet very clear, between the extracellular matrix and the nervous system and that this can modify neuroendocrine and neurological functions,
c) there are reasons for assuming that some chronic manifestations may derive from the activation of some kind of "vicious circle" mediated by the above interaction, to the extent that might result in some kind of neurophlogosis.

It can therefore be concluded, at least as an assumption, that the methods of physical medicine, activating peripheral stimulation mechanisms, can interact with the entire biological system. At this point it is necessary to specify what is meant by stimulation signal, and what the characteristics of the means through which translation of the said signal occurs are; it is therefore necessary to distinguish in particular, even though the distinction could be artificial, between biophysical effects and biochemical effects.

The biophysical effects, at least at macroscopic levels, relate to the state of the extracellular matrix, which normally in a sol phase tends to change into a gel phase in the presence of inflammation. Oedema appears to be substantially an anomaly in the extracellular matrix which has changed from the sol phase to the gel phase. The change of state is likely to be caused by a "stretching" of the matrix proteins (proteoglycanes and glycosaminoglycanes) with the consequent adhesion of layers of molecules of water forming the gel. But the change of state has the feature of taking place in a non-linear fashion under particular physical conditions of pressure, temperature, pH, etc.

The biochemical effects are strictly connected with cell metabolism, which takes place in a cyclical fashion, involving a cellular "respiratory chain" and providing for a continuous system of energy exchanges. This is the energy necessary to activate an ionic or covalent bond for the functions of protein oxidation/reduction, as well as the energy deriving from the reaction. It should not be forgotten that in general oxidation reactions give out energy while reducing reactions absorb energy.

The energy is absorbed or given out in the form of photons, which as experience and theoretical calculations confirm are characterized by wavelength, mainly in the visible/infrared region.

The energy cycle involving mitochondria is based on oxidative phosphorylation resulting in the formation of adenosine triphosphate (ATP) in the context of the citric acid cycle, correlated with the catabolism of proteins. The chain thus gives up energy and reduces oxygen with the formation of molecules of water. It is interesting to note that:
a) the wavelengths of the photons emitted and absorbed in the oxidation-reduction cycle of matrix proteins lie between 600 and 700 nm,
b) the rhythm of these exchanges has a frequency of between 50 and 200 Hz (the period for the transport of the electrons has been measured to be in the band of between 5 and 20 msec,
c) the rhythm of the cell respiratory chain, activated by the energy made available by the matrix protein oxidation-reduction cycle, has a typical frequency of the order of 1 Hz.

It must be emphasized that although on the one hand the emission of photons from the cell's respiratory chain represents a kind of "wastage", it also constitutes an optimized optical communications channel for all purposes. A complex series of experiments, including among others the work of F.A. Popp, has confirmed that the emission is consistent and is therefore capable of "synchronizing" the metabolic activities of adjacent cells; in particular the experiments have confirmed that the extracellular matrix responds to stimulation in the same way as that which occurs within a "window" of very particular energy, power and frequency, similar to what was demonstrated by Adey for the possibility of interference between electromagnetic fields and brain tissues. This of course provided that these cells function correctly. In other words pathological cells may not respond to stimulation and may interrupt - or modify - the regular flow of information in the photon optical channel.

The object of this invention is to provide an instrument capable of providing stimulation at very low energy which is capable of interacting with the biological system, imitating the "biological signal" produced by the metabolism of healthy tissues.

From US-A-4 232 678 a laser therapy equipment is known comprising a central control unit, an applicator handle, infra-red laser diode emission means located in an applicator handle. The said equipment does not emit a square wave but an impulse generated by a monostable oscillator activated by a square wave control.

From DE 3226507 a laser therapy equipment is disclosed comprising signal generating means, means for modulating the signal generated and laser emission means located in a applicator handle.

From DE 296 13 714 U a device is known which is concerned with the modulation of sequences of pulses and not with the modulation of square waves with variable Duty Cycle.

This invention therefore relates to equipment for laser treatment comprising signal generating means, means for modulating the signal generated, a central control unit and laser emission means, characterized in that the said modulated signal is derived from square wave modulation at a frequency of between 50 and 200 Hz combined with square wave modulation at a frequency of between 0.1 and 20 Hz.

In a preferred embodiment the said laser emission means comprise a laser diode which emits at a wavelength of between 250 and 1100 nm, and preferably between 600 and 700 nm. The diode peak potential lies between 1 and 5 mW square wave modulated at the primary frequency of 50-200 Hz with a duty cycle of not more than 10% and square wave super modulated at the secondary frequency of 0.1 - 20 Hz with a duty cycle of between 1% and 50%, with the provision that the stimulation time for an individual point can vary between 5 and 20 seconds.

Further advantages and features of the equipment according to this invention will be apparent from the following description of an embodiment thereof provided by way of a non-restrictive example with reference to the single plate of appended drawings, in which:

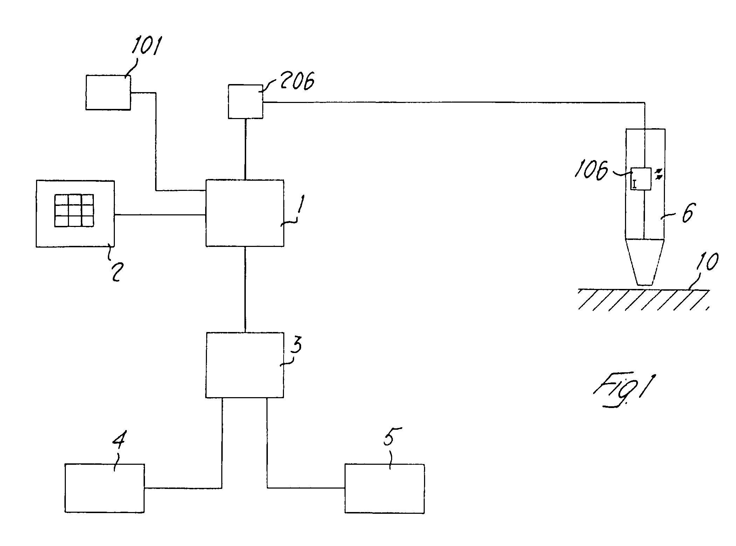

Figure 1 is a diagram showing an embodiment of the equipment according to this invention.

The equipment according to this invention is illustrated diagrammatically in the figure. 1 indicates the central control unit, to which are connected the two square wave signal generators 4 and 5 through mixer 3. Also connected to central unit 1 are: the periphery 2 of the operator interface, in this case a keyboard, delay/counter 101 and amplifier 206 which passes the modulated signal to laser diode 106 located in handle 6 positioned close to area 10 requiring treatment.

Operation of the equipment according to the invention will be obvious from what follows. The laser diode used emits radiation whose wavelength lies within the range between 250 and 1100 nm, and preferably between 600 and 700 nm, an interval which corresponds to the wavelength of the photons released in the biological system's oxidation reactions. The signal feeding diode 106 is modulated both to reduce its mean power and above all to imitate the biological signal produced by metabolism in healthy tissues. Modulation is carried out using the mixed signal of two square wave generators 4 and 5, which respectively produce a "carrier signal" of a frequency between 50 and 200 Hz, which permits interaction with the oxidative/reducing mechanisms characteristic of metabolic reactions, and a "modulating signal" of a frequency between 0.1 and 20 Hz which permits interaction with the body's biological rhythms. In the first case the duty cycle will be between 0.1 and 10%, in the second case between 1 and 50%.

The source cross-section of the light beam is of the order of 2-5 mm, and preferably 3 mm, with the focus of the beam located approximately 35 mm from the tip of applicator handle 6. The application distance may as a consequence vary between 35 and 100 mm from the tip of the handle. The energy dose, that is the quantity of energy administered, is determined by the stimulation time, which may vary between 5 and 20 seconds. The peak potential of the laser diode is normally between 1 and 5 mW.

With regard to the variable distance between the handle and the skin requiring treatment, the surface area of the cross-section of the beam on the skin may vary between 0.8 and 80 mm2. The mean power emitted by the diode depends on the peak power and the duty cycles of the signals used for modulation, and may adopt values between 0.0001 and 0.25 mW, and preferably of the order of 0.015 mW. As a consequence the incident energy, or as defined above the energy dose, deriving from the value of the mean power emitted as a function of application time lies within the range between 0.005 and 5 mJ, and preferably of the order of 0.15 mJ for each point stimulated.

The function of the equipment according to the invention is also defined by another two characteristic parameters, the calculation of which is based on the quantities described above. The first reference value is the flux, that is the power emitted per unit surface area, which in the case in point may therefore vary between 0.0000125 and 31.25 mW/cm2, and preferably of the order of 0.15 mW/cm2, while the second is the energy density, that is the incident energy per unit surface area, which is here of the order of 0.000625 to 625 mJ/cm2, and preferably 1.5 mJ/cm2.

From what has been stated above it is clearly apparent that the radiation emitted has characteristics, above all from the energy point of view, which are absolutely different from those typically used in equipment for laser treatment of the type known in the state of the art. In fact the energy values provided per unit surface area are usually very much higher, and may vary from approximately 1 J/cm2 in the treatment of open wounds to 100 J/cm2 in the treatment of severe pain. This type of treatment must necessarily make a balance between damage and benefit which is not always favourable for the patient.

Vice versa, the equipment according to this invention makes it possible to impart stimulation of virtually zero energy in order to elicit a systemic response through cybernetic mechanisms rather than energy mechanisms. In practice the radiation emitted through the equipment according to the invention should make it possible to "realign" the functioning of the dysmetabolic cells with that of the healthy cells, acting on the basis of impulses which do not substantially alter the energy balance of the biological system subjected to treatment.

Advantageously the equipment according to this invention may be provided with several modules emitting coherent radiation, which can be activated as alternatives to each other, in order to cover a wider spectrum of emitted radiation. Operator interface periphery 2 may also comprise a topographical anatomical map, and a module for locating sensitive points, that is the so-called "acupuncture points" known in the practice of reflexology.

## Claims

1. Laser therapy equipment, comprising a central control unit (1), an applicator handle (6), laser emission means (106) located in an applicator handle (6), two square-wave signal generating means (4,5), means for generating a modulation signal (3) for modulating the laser emission means by combining two square-wave signals from said square-wave signal generating means, where one square-wave signal is at a frequency between 50 and 200 Hz and the other square-wave signal is at a frequency between 0.1 and 20 Hz

2. Equipment according to claim 1, in which said laser emission means comprises a laser diode emitting at a wavelength of between 600 and 700 nm.

3. Equipment according to claim 2, in which the peak power of the diode lies between 1 and 5 mW.

4. Equipment according to any one of claims 2 to 3, in which the source cross-section of the laser beam emitted is of the order of 2-5 mm, and preferably 3 mm, with the focus of the beam located approximately 35 mm from the tip of the applicator handle (6).

5. Equipment according to any one of preceding claims 2 to 4, in which the mean power emitted by the diode is between 0.0001 and 0.25 mW.

## Patentansprüche

1. Lasertherapieeinrichtung mit einer zentralen Steuereinheit (1), einem Applikatorgriff (6), einem Laseremissionsmittel (106), das in einem Applikatorgriff (6) angeordnet ist, zwei Rechteckfunktionssignalerzeugungsmitteln (4, 5), einem Mittel zum Erzeugen eines Modulationssignals (3) zum Modulieren des Laseremissionsmittels durch Kombinieren der zwei Rechteckfunktionssignale von den Rechteckfunktionssignalerzeugungsmitteln, wobei ein Rechteckfunktionssignal bei einer Frequenz zwischen 50 und 200 Hz liegt und das andere Rechteckfunktionssignal bei einer Frequenz zwischen 0,1 und 20 Hz liegt.

2. Einrichtung nach Anspruch 1, in der das Laseremissionsmittel eine Laserdiode aufweist, die bei einer Wellenlänge zwischen 600 und 700 nm abstrahlt.

3. Einrichtung nach Anspruch 2, in der die Spitzenleistung der Diode zwischen 1 und 5 mW liegt.

4. Einrichtung nach Anspruch 2 oder 3, in welcher der Quellenquerschnitt des abgestrahlten Laserstrahls in der Größenordnung von 2 bis 5 mm liegt und vorzugsweise bei 3 mm, wobei der Brennpunkt des Strahls ungefähr 35 mm von der Spitze des Applikatorgriffs (6) entfernt liegt.

5. Einrichtung nach einem der vorhergehenden Ansprüche 2 bis 4, in der die mittlere Leistung, welche von der Diode abgestrahlt wird, zwischen 0,0001 und 0,25 mW liegt.

## Revendications

1. Equipement de traitement au laser, comprenant une unité de commande centrale (1), une poignée d'applicateur (6), un dispositif d'émission du laser (106) situé dans la poignée de l'applicateur (6), deux dispositifs pour générer des signaux carrés (4, 5), des dispositifs pour générer un signal de modulation (3) pour moduler le dispositif d'émission du laser en combinant deux signaux carrés provenant dudit dispositif de génération de signaux carrés, dans lequel un signal carré a une fréquence entre 50 et 200 Hz et l'autre signal carré une fréquence entre 0,1 et 20 Hz.

2. Equipement selon la revendication 1, dans lequel ledit dispositif d'émission de laser comprend une diode-laser émettant à une longueur d'onde entre 600 et 700 nm.

3. Equipement selon la revendication 2, dans lequel la puissance de crête de la diode est entre 1 et 5mW.

4. Equipement selon une quelconque des revendications 2 à 3, dans lequel la coupe transversale du faisceau laser émis est de l'ordre de 2-5 mm, et de préférence 3 mm, avec la concentration du faisceau située à environ 35 mm de la pointe de la poignée de l'applicateur (6).

5. Equipement selon une quelconque des revendications précédentes 2 à 4, dans lequel la puissance moyenne émise par la diode est entre 0,0001 et 0,25 mW.
